(19) **Europäisches Patentamt / European Patent Office / Office européen des brevets**

(11) **EP 2 477 587 B1**

(12) **EUROPEAN PATENT SPECIFICATION**

(45) Date of publication and mention of the grant of the patent:
**11.01.2017 Bulletin 2017/02**

(51) Int Cl.:
*A61F 9/008* (2006.01)  *A61F 9/009* (2006.01)

(21) Application number: **10760531.3**

(22) Date of filing: **16.09.2010**

(86) International application number:
**PCT/US2010/049177**

(87) International publication number:
**WO 2011/035063 (24.03.2011 Gazette 2011/12)**

(54) **REGISTRATION OF CORNEAL FLAP WITH OPHTHALMIC MEASUREMENT AND/OR TREATMENT DATA FOR LASIK AND OTHER PROCEDURES**

REGISTRIERUNG EINER HORNHAUTKLAPPE DURCH OPTISCHE MESS- UND/ODER BEHANDLUNGSDATEN FÜR LASIK- UND ANDERE VERFAHREN

ENREGISTREMENT D'UN VOLET CORNÉEN AVEC MESURE OPHTALMIQUE ET/OU DES DONNÉES DE TRAITEMENT POUR LASIK ET AUTRES PROCÉDURES

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **18.09.2009 US 243654 P**

(43) Date of publication of application:
**25.07.2012 Bulletin 2012/30**

(73) Proprietor: **AMO Development, LLC**
**Santa Ana, CA 92705 (US)**

(72) Inventors:
• **STEVENS, Julian**
**Kenwood London N64JS (GB)**
• **CHERNYAK, Dimitri**
**Sunnyvale, CA 94086 (US)**
• **ZICKLER, Leander**
**Mountain View, CA 94041 (US)**

(74) Representative: **Hoffmann Eitle**
**Patent- und Rechtsanwälte PartmbB**
**Arabellastraße 30**
**81925 München (DE)**

(56) References cited:
**WO-A2-2009/059251  US-A1- 2003 223 037**
**US-A1- 2005 024 586  US-A1- 2009 069 794**

**Description**

CROSS REFERENCE TO RELATED APPLICATION DATA

The present application claims the benefit under 35 USC 119(e) of US Provisional Application No. 61/243,654 filed September 18, 2009.

BACKGROUND OF THE INVENTION

The present invention pertains generally to ophthalmic surgery which is useful for correcting vision deficiencies. More particularly, the present invention pertains to the incising of corneal tissues, optionally for the formation of corneal flaps and the like used in ophthalmic surgery.

Corneal shape corrective surgeries are commonly used to treat myopia, hyperopia, astigmatism, and the like. Laser refractive procedures employing an excimer laser include LASIK (Laser Assisted In-Situ Keratomileusis), PRK (Photo Refractive Keratectomy) and LASEK (Laser Subepithelial Keratomileusis).

An example of such a system is given in US 2003/0223037.

During LASIK, a suction ring is typically placed against sclera tissue (the white part of the eye) to hold an interface firmly against the eye. In some embodiments, a surgeon then uses a microkeratome with an oscillating steel blade to make a partial cut through a front surface of a cornea. The microkeratome automatically passes the blade through the cornea so as to create a thin flap of clear tissue on the front central part of the eye. Such microkeratomes are mechanical devices that use an automated blade to create a flap. The suction ring is then removed, and the flap is lifted back to expose stromal tissue for ablation with the excimer laser. More recently, femtosecond laser systems have been developed to form laser incisions in the corneal tissue so as to form the corneal flap. The excimer laser can be programmed to correct a desired amount of visual defect by directing a beam of laser energy onto the exposed stromal tissue, the beam typically comprising a series of laser pulses. Each pulse removes a very small and precise amount of corneal tissue so that the total removal of stromal tissue alters and corrects the refractive properties of the overall eye. After irrigation with saline solution, the corneal flap is folded back to adhere to the underlying stromal tissue.

Currently, physicians estimate the centering of the microkeratome or femtosecond laser incision in the cornea to create the corneal flap. If the center is not correct, the resulting vision from ophthalmic surgery may not be able to proceed, or may not achieve the desired refractive improvements. To help ensure that the surgery can proceed, flaps that are significantly larger than the planned underlying refractive reshaping can be used. While generally safe and effective the use of oversized corneal flaps may not be ideal for all patients. Similarly, while skilled physicians may routinely position the flaps with sufficient accuracy, patients with unusual needs may benefit from improved techniques.

In light of the above, it would be desirable to provide systems and methods for forming incisions in an eye, particularly for accurately locating a corneal flap for vision correcting procedures.

BRIEF SUMMARY OF THE INVENTION

The present invention is described exclusively by the claims which follow. In particular surgical methods are only described for illustrative purposes. The present invention generally provides improved systems and devices for forming an incision in an eye of a patient. In exemplary embodiments, the invention provides improved systems for forming a corneal flap so as to expose stroma underlying a corneal epithelium in preparation for LASIK or other refractive corneal procedures. The desired sizing and centering of the corneal flap may be determined with reference to a first image of the eye obtained during wavefront aberrometer measurements, topographic measurements, or other diagnostic procedures. Rather than relying on physician positioning of a suction interface against an eye to determine the location of the flap, a second image of the eye may be obtained when the eye is prepared for formation of the incision, optionally after the interface is in place. In alternative embodiments, the second image may be obtained just before engaging the eye with the interface, or as the interface engages the eye. The second image may be acquired through a clear surface of an applanation lens of the interface overlying the cornea. Image processing techniques can compare the first and second images of the eye, allowing the desired flap to be registered to the eye, typically by identifying X-Y offsets between the images, rotational offsets between the images, and/or the like. Such techniques are particularly well suited to using femtosecond or other intrastromal lasers for incising the cornea.

In one aspect, the invention provides a system for performing surgery on an eye.

The eye has a cornea, and the system may perform a method that comprises capturing a first image of the eye during a diagnostic procedure and determining a desired corneal incision referenced to the first image. A second image of the eye is captured. The first and second images are processed so as to generate corneal incision location information referenced to the second image. The cornea is incised so as to form the desired corneal incision using the incision location information.

In many embodiments, the desired incision will define a corneal flap, typically so as to allow an endothelial layer of the cornea to be temporarily displaced and expose underlying stroma. A flap geometry of the corneal flap can be determined in response to a planned corneal refractive correction. For example, the eye may be measured by a wavefront aberrometer, a topographer, or the like. A corneal refractive correction may be determined based on these or other diagnostic procedures, with an appropriate overall ablation profile generated to provide a smooth transition zone around the refractive correction. The flap geometry may then be determined based on the ablation profile, with the size of the flap being sufficiently large that the ablation profile remains within stromal tissue, the location of the flap being positioned and centered appropriately over the ablation profile, and the flap hinge or uncut tissue region being appropriately oriented for the treatment system, physician preferences, and the like. While many corneal flaps may be substantially circular in shape, flap geometries which are non-circular may also be used. For example, when an astigmatic patient would benefit from an elongate ablation profile, an elliptical or other non-circular flap geometry may be determined. This desired flap geometry and location may be referenced to the first image.

The patient may be moved between the diagnostic measurement and the incision, or may remain in the same location. Even when the patient does not move, sufficient time will typically pass between the measurement and the incision for the eye to move significantly between the first and second images. Regardless, the images may be processed by comparing the first image with the second image using image processing techniques so as to register the desired corneal incision from the first image to the second image. This registration may, for example, rely on a center of the iris in the first image and a center of the iris in the second image and the target flap location information may include an X-Y offset. In other embodiments, the processing may allow torsional registration of the desired corneal flap from the first image to the second image based on iris features in the first image and corresponding iris features in the second image, with the flap location information including an angular offset or the like.

The incising of the cornea typically is performed by directing femtosecond laser energy toward the eye, although other embodiments may employ other instrastromal lasers, mechanical cutting devices, or the like. In many embodiments, an interface will be affixed to the eye by suction while generating the second image. The interface may include a transparent surface disposed over the cornea during use so that the second image is obtained by imaging the eye through the transparent surface of the interface. A refractive laser treatment may be generated in response to the diagnostic procedure, and a third image of the eye (typically an image associated with a refractive reshaping laser system) may be acquired to facilitate registering the refractive treatment with the third image.

Optionally, a registered corneal flap may be used to facilitate tracking of the eye during refractive correction. For example, a refractive laser treatment may be generated in response to the diagnostic procedure. A third image of the eye may be captured, with the third image associated with a refractive reshaping laser system. The refractive treatment can then be registered with the third image, for example, by aligning a laser treatment center to the registered corneal flap geometry. More specifically, the desired corneal incision may define a desired corneal flap having a desired flap center and a desired rotationally asymmetric feature, with both being referenced to the first image. The actual corneal incision then defines an actual corneal flap having an actual flap center and an actual rotationally asymmetric feature. A third image of the eye is captured, and the registration of the actual flap geometry with the eye may optionally be verified to be within a desired threshold (and/or appropriate offsets between the actual and desired flap geometry may be determined) by a comparison of an iris center and iris features in the third image to the actual flap center and the actual asymmetric flap feature in the third image. Registering of the refractive treatment with the third image (and/or subsequent images) may be provided by determining an X-Y offset between the desired flap center referenced to the first image and the flap center in the third image, and determining an angular offset between a desired flap angle referenced to the first image and the rotationally asymmetric feature in the third image.

In another aspect, the invention provides a system capable of executing a method of performing surgery on an eye.

The eye has a cornea, and the method comprises forming a flap in the cornea by incising the cornea. A first image of the eye is captured during a diagnostic procedure. A location of the flap referenced to the first image is determined, optionally by processing the image, using diagnostic data (such as wavefront and/or topographic data) and/or the like. A second image of the eye is captured, and the first and second images are processed so as to generate corneal treatment location information referenced to the second image. The cornea can then be treated (such as by directing an appropriate pattern of ablative laser energy to stroma exposed by displacing the flap) using the treatment location information.

In another aspect, the inventive system may execute a method of forming a corneal flap for laser surgery on an eye. The eye has a cornea, and the method comprises capturing a first image of the eye, the first image comprising image data. A reference location of the eye may be determined by processing the image data. A desired corneal flap may be determined with respect to the reference location in the first image, and the cornea may be incised so as to form the flap by registering the desired corneal flap geometry to the eye.

In yet another aspect, the invention provides a system for treating an eye having a cornea. The system comprises an ophthalmic diagnostic device having a first image capture device for obtaining a first image of the eye during a diagnostic procedure. A femtosecond laser system having a second image capture device obtains a second image of the eye during a procedure to form of a laser corneal incision. The corneal incision is referenced to the first image, and a processor system couples the diagnostic device to the laser system. The processor system directs laser energy from the laser system toward the eye during use by comparing the first image with the second image so as to register the corneal incision with the eye.

In another aspect, the inventive system may execute a method of performing surgery on an eye, the eye having a cornea. The method comprises capturing a first image of the cornea and determining a desired diagnostic procedure of the cornea referenced to the first image. The cornea is incised so as to form a corneal flap referenced to the first image. A second image of the cornea is captured encompassing the cornea flap, and the cornea is reshaped per the desired diagnostic procedure by directing the refractive correction to the cornea with reference to the corneal flap in the second image.

BRIEF DESCRIPTION OF THE DRAWINGS

FIG. 1 schematically illustrates a simplified system of one embodiment of the present invention;

FIG. 1A is a schematic perspective view of a refractive laser surgery system and patient support system, components of which may be modified for use with the system of FIG. 1;

FIG. 2A illustrates a first image of an eye taken with a measurement system;

FIG. 2B illustrates a second image of the eye taken with a treatment system;

FIG. 3 schematically illustrates one embodiment of registering a corneal flap for laser surgery on an eye;

FIG. 4 schematically illustrates another embodiment of registering a corneal flap for laser surgery on an eye;

FIG. 5 schematically illustrates a method of the registering a first image with a second image;

FIG. 6 is a simplified cross-sectional illustration of an ocular stabilization and applanation interface device showing operation of the device by engaging a transparent surface of the device against the cornea, imaging the eye through the transparent surface, and transmitting femtosecond laser energy through the transparent surface so as to form an incision in the cornea of an eye;

FIGs. 7A and 7B schematically illustrates an embodiment of an diagnosing and treating an eye by registering a corneal flap with the eye, and then directing refractive tissue removal and/or reshaping toward the eye with reference to the corneal incision location; and

FIGs. 8A-8C schematically illustrate exemplary formation of registered corneal incisions using a femtosecond laser system so as to form corneal flaps having flap geometries with rotationally asymmetric features that facilitate determining both X-Y offsets and angular offsets with reference to images of the corneal flaps.

DETAILED DESCRIPTION OF THE INVENTION

Cyclotorsional rotation of the eye and pupil center shift may occur between diagnosis or measurement of an eye and treatment of that eye. For example, corneal flap geometry determined using a diagnostic procedure may not be as accurate as desirable if applied without compensating for movement of the eye during a subsequent laser treatment procedure. The present invention recognizes and mitigates this problem by registering (or aligning) the corneal flap geometry and treatment information from the diagnostic procedure to the desired location on the cornea when incising the cornea so as to form the corneal flap. Additionally, the corneal flap incision may be used for alignment of the refractive correction (e.g., LASIK procedure). In various other embodiments, one or more corneal incisions (e.g., not necessarily for corneal flap forming) with a desired asymmetry may be used to register the treatment information from the diagnostic procedure to the desired location on the cornea. For example, a non-rotational symmetric corneal incision may be used to torsionally register the refractive correction to the cornea.

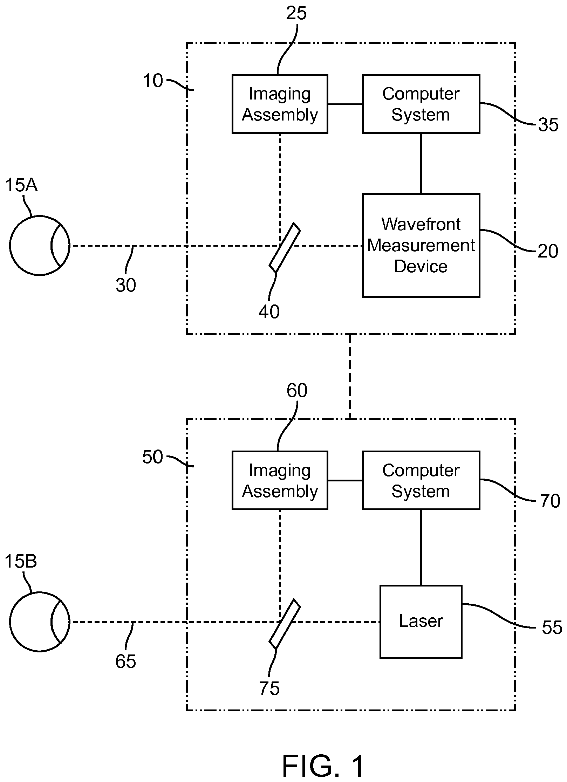

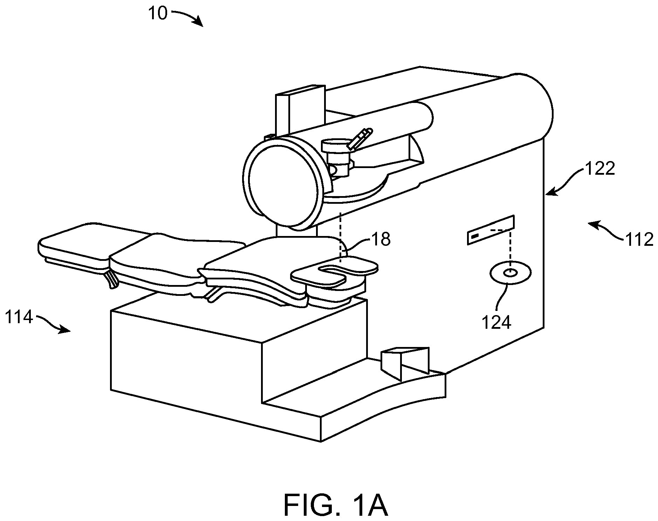

FIG. 1 schematically illustrates a simplified system of one embodiment of the present invention. The system includes a measurement device 10 used during a diagnostic procedure and a laser surgery system 50 used during a treatment procedure. The diagnostic procedure may be done at the same time as the treatment procedure, or it may precede the treatment procedure by minutes, hours, days or weeks. The measurement device 10 is capable of generating images of the eye 15 and of providing information helpful for determining a desired corneal flap geometry. The flap geometry will often be referenced to the image, so that a relationship between the location of the flap incision and the image data can be established. The corneal flap geometry is often linked to a feature or reference location on the eye 15 which can be identified in the image, such as a pupil center (located at the center of the inner iris boundary), the center of the outer iris boundary or limbus, natural markings included in the iris, visible limbal landmarks or features, and the like. Along with locating and/or determining the desired corneal flap geometry, the measurement device 10 may also include at least a portion of a processor system capable of calculating a set of treatment instructions to be used by a laser incision system, such as femtosecond laser system 50.

The exemplary measurement system 10 includes a wavefront measurement device 20, such as a wavefront aberrometer, and an imaging assembly 25. Imaging assembly 25 captures an image of the eye at substantially the same time (so that the eye does not move between the image and the measurement) that the wavefront measurement device 20 directs a beam 30 toward the eye of a patient in a diagnostic procedure under the direction of a computer system 35. Measurement device 20 and imaging assembly 25 may be optically coupled to optics 40, which directs a measurement beam 30 to the eye 15A, an image from the eye to the imaging assembly, and a measurement image from the eye back to the measurement device. The computer system 35 optionally determines a desired corneal flap geometry based on the images generated by the measurement system 10, often with the input of a system operator. The computer may store the corneal flap geometry, wavefront measurements and images of the patient's eye. One or more different incisions, a set of incisions or the like may be calculated for a desired corneal flap geometry. While the incisions are generally applied to form the desired corneal flap geometry, an individual incision or set of incisions may optionally be calculated to be formed in the cornea in other embodiments. As the wavefront measurement and image are substantially contemporaneous, and as the structures of the imaging assembly and the measurement device are optically and/or mechanically coupled, the location information included in the image and the measurement can be associated. In some embodiments, the computer processor 35 may also generate and save additional treatment information, such as an ablation profile or laser sculpting based on the image data that can later be downloaded into a refractive laser system 110 (see FIG 1A). Suitable measurement systems may include structures based on the WaveScan Wavefront® System commercially available from Abbott Medical Optics, Inc. (AMO) of Santa Ana, California, the Zyoptix® diagnostic workstation commercially available from Bausch and Lomb of Rochester, New York, and others.

The laser system 50 includes a laser 55, such as a femtosecond laser, and imaging assembly 60 that obtains an image of the eye. Images from imaging assembly 60 are substantially contemporaneous with the incision of the eye using laser 55, and imaging assembly 60 and laser 55 are mechanically and/or optically coupled together, so that the images from imaging assembly 60 can be used to help direct a laser beam 65 to the eye 15B of the patient during a treatment procedure under the direction of a computer system 70. Laser 55 and imaging assembly 60 may be optically coupled to optics 75, which directs beam 65 to the eye 15B. The computer system 70 will generally direct pulses of laser energy toward the cornea to form an incision in the cornea so as to form a flap of corneal tissue exposing the stroma underlying the corneal epithelium. Subsequent ablation or removal of the exposed stroma can alter the refractive characteristics of the eye. In some embodiments, the ablation profile generated with the measurement system 10 will be downloaded into computer system 70, and the corneal correction may be performed using the femtosecond laser 55. Suitable femtosecond laser systems may include the iFS™ Advanced Femtosecond Laser system commercially available from AMO.

Referring now to FIG. 1A, some embodiments may be incorporated into, and/or may be used with a laser eye surgery system 110. Laser eye surgery system 110 generally includes a laser system 112 and a patient support system 114. Laser system 112 includes a housing that contains both a laser and a system processor 122 having software 124. The laser generates an excimer laser beam 18, which is directed to a patient's eye under the direction of a system operator. Delivery optics used to direct the laser beam, the microscope mounted to the delivery optics, and the like may employ existing structures from commercially available laser systems, including the STAR S4 IR® excimer refractive laser systems available from AMO. This exemplary refractive laser system includes an imaging system to laterally and torsionally register the eye with a planned refractive treatment, and to track (laterally and/or torsionally) the eye during the treatment so that the desired refractive change is accurately produced in the eye without having to rigidly restrain the eye. Suitable tracking systems for use in laser system 110 include those described in U.S. Patent No. 6,322,216, entitled "Two Camera Off-Axis Eye Tracker for Laser Eye Surgery," and suitable torsional and lateral registration and tracking systems include those described in U.S. Patent No. 7,044,602, entitled "Methods and Systems for Tracking a Torsional Orientation and Position of an Eye," the full disclosures of both of which are incorporated herein by reference. Processor 122 may be included in a processor system that helps transfer data between and/or provide control over a diagnostic, incising, and refractive treatment system including measurement system 10, laser system 50, and laser system 110.

Computer systems 35, 70, and 122 may comprise hardware and/or software, often including one or more programmable processor unit running machine readable program instructions or code for implementing some or all of one or more of the methods described herein. The code will often be embodied in a tangible media such as a memory (optionally a read only memory, a random access memory, a non-volatile memory, or the like) and/or a recording media (such as a floppy disk, a hard drive, a CD, a DVD, a memory stick, or the like). The code and/or associated data and signals may also be transmitted to or from the processor via a network connection (such as a wireless network, an Ethernet, an internet, an intranet, or the like), and some or all of the code may also be transmitted between components of the system and within processor via one or more bus, and appropriate standard or proprietary communications cards, connectors, cables, and the like will often be included in the processor. The processor will often be configured to perform the calculations and signal transmission steps described herein at least in part by programming the processor with the software code, which may be written as a single program, a series of separate subroutines or related programs, or the like. The processor may comprise standard or proprietary digital and/or analog signal processing hardware, software, and/or firmware, and will typically have sufficient processing power to perform the calculations described herein during treatment of the patient, the processor optionally comprising a personal computer, a notebook computer, a tablet computer, a proprietary processing unit, or a combination thereof. Standard or proprietary input devices (such as a mouse, keyboard, touchscreen, joystick, etc.) and output devices (such as a printer, speakers, display, etc.) associated with modern computer systems may also be included, and processors having a plurality of processing units (or even separate computers) may be employed in a wide range of centralized or distributed data processing architectures.

The image data, desired flap geometry and/or the customized ablation profile may be transferred from measurement system 10 to femtosecond system 50 through a computer readable medium or through direct connection 80, such as a local or wide-area network (LAN or WAN). Measurement system 10 and/or treatment system 50 can have software stored in a memory and hardware that can be used to control the taking of images and delivery of flap cutting or ablative energy to the patient's eye, the location or the position (optionally including translations in the x, y, and z directions and torsional rotations) of the patient's eye relative to one or more optical axes of the imaging assemblies, and the like. In exemplary embodiments, among other functions, measurement system 10, laser system 50, and/or refractive laser treatment system 110 can be programmed to calculate treatment or ablation profiles based on the image(s) taken with measurement system 10 and the image(s) taken by treatment system 50, and measure the offset between the patient's eye in the two images. Additionally, treatment systems 50 and 110 can be programmed to measure, effectively in real-time, the movement or position x(t), y(t), z(t), and rotational orientation of the patient's eye relative to the optical axis of the laser beam so as to allow the computer system 70 to register or align the desired corneal flap geometry on the real-time position of the patient's eye.

The measurement system 10 may also calculate a treatment plan or corneal ablation pattern for ablating the eye with treatment system 50 so as to correct the optical errors of the eye. Such calculations will often be based on both the measured optical properties of the eye and on the characteristics of the corneal tissue targeted for ablation (such as the ablation rate, the refractive index, and the like. The results of the calculation will often comprise an ablation pattern in the form of an ablation table listing ablation locations, numbers of pulses, ablation sizes, and or ablation shapes to effect the desired refractive correction. Such a treatment table can then be transmitted to refractive treatment system 110 for refractive correction of the eye.

In order to register the desired corneal flap geometry of the patient's eye during the treatment, the images from the patient's eye taken by the measurement system 10 and treatment system 50 should share a common coordinate system. The common coordinate system may be based a center of the pupil or inner iris boundary, a center of the outer iris boundary, limbal landmarks, iris features or striations included in the iris, artificial landmarks or markings imposed on the eye, or any other suitable feature of the eye. The desired corneal flap geometry may be positionally and torsionally aligned from the measurement system 10 to the treatment system 50 using the common coordinate system.

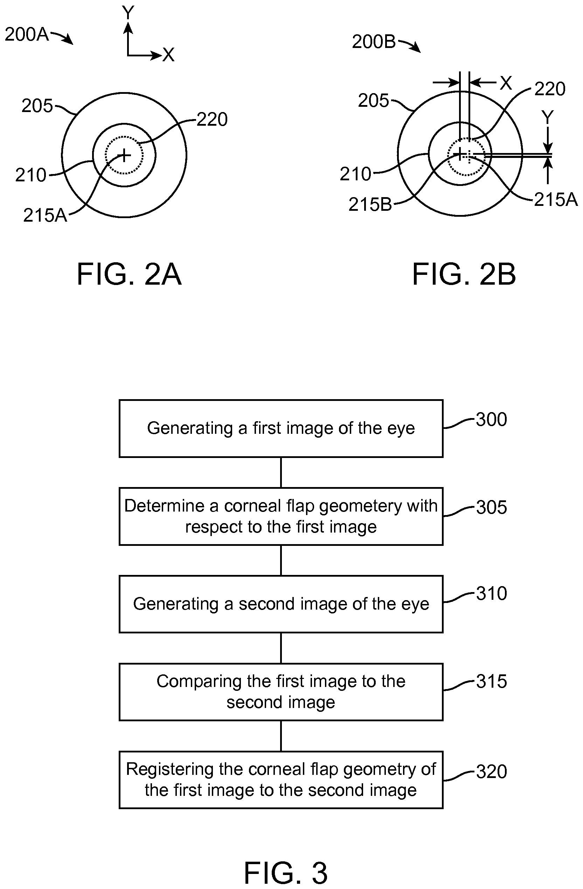

FIGs. 2A and 2B schematically illustrate a camera view obtained at the time of wavefront acquisition and a view obtained at the time of femtosecond flap creation. The imaging assemblies will typically include an image capture device in the form of a digital image sensor such as a charge-coupled device (CCD) or the like. Hence, the captured images will often be transmitted from the imaging assemblies to the processors as digital pixel data. Image processing software of the processor system analyses this digital data to determine the location of the centroid of the pupil or center of the limbus in the image from measurement device 10 when the wavefront is obtained, and the same centroid is determined to identify the desired target at the time of LASIK flap femtosecond laser application. This provides a clear advantage in avoiding decentered LASIK flaps and promoting a perfectly centered flap each time. For image capture and analysis of incisions, it may be desirable to provide high contrast visualization/detection of the incisions, for example, to detect refractive index changes, incision edge features, polarization changes, and the like.

FIG. 2A illustrates a first image 200A of an eye 205 taken with measurement system 10, the first image 200A includes a pupil 210 and a reference location 215A. A refractive prescription is determined from the measurement data obtained by wavefront measurement device 20, so that a size of the corneal flap can be determined that is sufficient to encompass the ablation profile associated with the refractive prescription. Any of a wide variety of known techniques can be employed to determine the refractive prescription, ablation profile, an associated ablation shot pattern for the refractive laser, and the like, based on the wavefront data, including those used in the commercially available systems identified above. The computer systems 35 and/or 70 of measurement system 10 and/or treatment system 50 determine a suitable corneal flap geometry based on the refractive prescription, physician preferences (as input into the computer system), and the like. The flap geometry may include a flap center, flap size, flap hinge orientation, flap shape, and/or the like. The flap size will typically be sufficiently large to expose stroma throughout an area of the refractive reshaping, so that the flap will generally be larger than the associated refractive prescription. Where the prescription area is non-circular, the flap may have an elliptical or other non-circular shape.

As the flap geometry is determined with reference to diagnostic data associated with first image 200A, the flap center or other reference location 215A, flap hinge orientation, and other flap geometry will similarly be referenced to the tissue of the eye as shown in the first image 200A. Note that the reference location 215A and first image 200A are schematic representations of the location images and data that may be used. In many embodiments, a time series of wavefront data (and associated images) may be obtained. Similarly, one or more topographical measurements (including associated image and shape data) may be acquired. The final prescription may be derived by registering and combining this information. Similarly, additional reference locations may be identified in the eye, particularly when it is desired to torsionally register the flap to the eye tissue, with iris features or the like often being imaged and used. FIG. 2B shows a second image 200B of the eye 205 taken with treatment system 50. The second image 200B includes the pupil 210 and a reference location 215B. A comparison of the two images shows that the patient's eye has moved and the reference locations 215A and 215B are not coincident. The treatment system 50 can correct by adding in a translation measurement (X-Y) and/or torsional alignment to position the flap geometry at the proper reference location.

FIG. 3 schematically illustrates one embodiment of registering a corneal flap for laser surgery on an eye. An initial step in the method is to generate a first image of the eye (Step 300), which is done with a measurement system during a diagnostic procedure. Generating the first image may include measuring the eye with a wavefront aberrometer. A corneal flap geometry is determined with respect to the first image (Step 305). A set of laser instructions may also be determined for cutting the corneal flap during treatment. Optionally, a set of treatment instructions may be calculated for a treatment laser. A second image of the eye is generated (Step 310), which is done by a treatment system prior to, or during, a treatment procedure. Generating the second image of the eye may include measuring the eye with a femtosecond laser. The first image and second image are compared (Step 315). The corneal flap geometry of the first image is then registered to the second image (step 320). Registering the corneal flap geometry of the first image to the second image may include aligning a center of the pupil in the first image to a center of the pupil in the second image. The registration may include an X-Y offset from a center of the pupil in the second image. The registration may also include rotational registration.

Since the first and second images of the eye contain the pupil and iris, in some embodiments it may be more accurate to register the images by calculating the center of the pupil and the center of the outer iris boundary and expressing the position of the pupil center with respect to the center of the outer iris boundary. The center of the outer iris boundary may be described as a center of a circle corresponding to the outer boundary of the iris and may be located using an iris finding algorithm. The position of the center of the inner iris boundary or pupil may be compared to the outer iris boundary to calculate an offset from the outer iris center.

One embodiment of the invention may be implemented in the following manner:

1) A patient's eye is measured with a wavefront aberrometer and a desired corneal flap geometry is determined. In addition, a treatment is calculated as a set of instructions for a laser, such as an excimer. A first image of the eye is taken during the measurement to serve as a reference for subsequent treatment registration to the corneal position under the laser.

2) The diagnostic information may then be loaded into a femtosecond laser used to create the corneal flap. The information may be loaded into the laser by networking software or a data transfer device, such as a USB disk. A second image of the eye may be taken with a similar camera, similar illumination, similar field of view, and similar magnification.

3) Image processing software will analyze the first image and the second image to determine preferred flap center location and pass the coordinates to the femtosecond laser.

4) After the corneal flap is cut, the patient is transferred to an excimer laser for the refractive portion of the LASIK procedure. The laser may take another image (third image) of the eye as part of an iris registration process to align the treatment center of the laser to the same position as the center of the wavefront measurement and the flap center.

As one alternative, the entire refractive procedure may be carried out on the femtosecond laser by either selective cutting of corneal tissue to induce corneal shape changes (including but not limited to AKs, LRIs, RKs) or by removing the volume of material corresponding to the "tissue lens" required to achieve required refractive target. Such removal can be achieved by cutting the stroma at the targeted depth across the entire treatment zone and then physically lifting the tissue above the cut surface. In either case, using iris based registration will ensure the correct placement of the treatment with respect to the cornea and the flap.

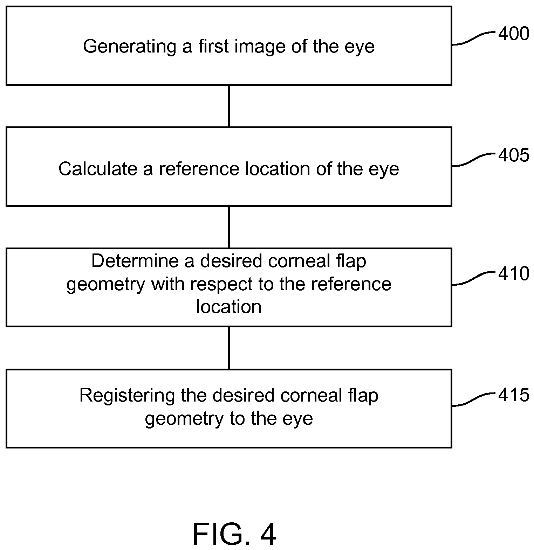

FIG. 4 shows another embodiment of the invention that may be implemented in the following manner:

1) A first image of the eye is taken during the measurement wavefront aberrometer to serve as a reference for subsequent treatment registration to the corneal position under a laser (Step 400).

2) A reference location of the eye is calculated (Step 405). The reference location may be a pupil center, the center of the iris boundary or limbal landmarks.

3) A desired corneal flap geometry is determined with respect to the reference location (Step 410). Optionally, a treatment may be calculated as a set of instructions for a laser.

4) The desired corneal flap geometry is registered to the eye (Step 415). The flap may be cut with a femtosecond laser. After the corneal flap is cut, an excimer laser may be used for the refractive portion of the LASIK procedure. The laser may take another image of the eye to align the treatment center of the laser to the reference location and the flap center.

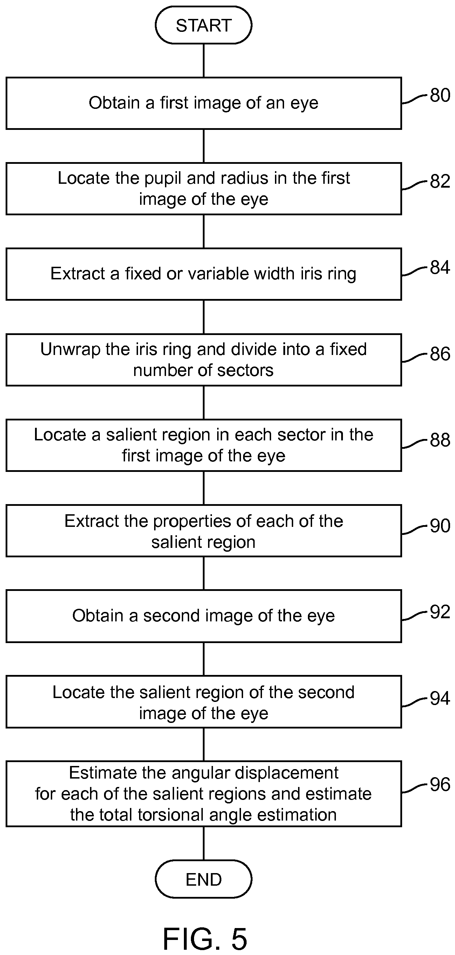

FIG. 5 schematically illustrates the data flow through an alignment process that can torsionally register a reference image with a second image of the eye to determine the torsional displacement between the two images of the eye. An initial step in the method is to obtain the first, reference image. (Step 80). In one embodiment, the first or reference image is a grayscale image of the patient's eye that is taken by a CCD camera in the wavefront measurement device under infrared illumination ($\lambda$ = 940 nm). The image contains the pupil and the iris. In some images, part of the iris may be occluded by one or both of the eyelids or may be cropped by the camera's field of view.

A pupil finding algorithm can be used to locate the pupil, calculate the radius of the pupil and find the center of the pupil. (Step 82). In one embodiment the pupil is located by thresholding the image by analyzing a pixel value histogram and choosing the position of a first "dip" in the histogram after at least 2000 pixels are below the cutoff threshold. All pixels below the threshold are labeled with "1" and pixels above the threshold are labeled with "0". Pixels labeled with "1" would generally correspond to the pupil, eyelashes, and possibly other regions of the image.

If desired, the selected pupil region can be filled to remove any holes created by reflections, or the like. Optionally, in some embodiments an iris finding algorithm can be used to locate the iris, calculate the radius of the iris, and/or locate the iris center. In embodiments of the present invention, the boundary of the iris may be localized with subpixel accuracy, but it might be slightly displaced from its true location if the shadows in the image soften the boundary edge.

Next, after the pupil center (and/or iris center) are located, a width of the iris ring can be extracted from the images. (Step 84). The iris can be treated as an elastic sheet stretched between pupil and the outer rim of the iris. In embodiments that do not use the iris finding algorithm, the width of the iris band can be set to or based on whether the patient has dark-colored eyes or light-colored eyes, or as being roughly constant for all people. The iris ring can then be unwrapped and divided into a fixed number of sectors, by converting the Cartesian iris coordinates into polar coordinates, centered at the pupil. (Step 86). In alternative embodiments, it may be possible to analyze the iris ring without unwrapping it. In some embodiments, the iris ring can be sampled at one-pixel steps in the radial direction for the reference image. Optionally, to reduce aliasing, the images can be smoothed with $\sigma$ = 1 pixel Gaussian kernel.

Optionally, the dynamic range of pixel values in the iris may be adjusted to remove outliers due to reflections from the illumination LED lights. The pixel value histogram can be thresholded so that all the pixels with values above the threshold are assigned the value of the threshold. Also, some band-pass filtering may be applied to the iris bands prior to region selection to remove lighting variation artifacts.

After the iris is divided into sectors, one salient region or marker in each sector in image can be located and its properties can be extracted. (Steps 88, 90). In some embodiments, the iris region is segmented into twenty four sectors of fifteen degrees.

The markers in the reference image can be stored and later located in the second image of the eye so as to estimate the torsional displacement of the eye between the two images. The markers should be sufficiently distinct and have high contrast. There are several possible ways to select such points. In one implementation, a square mask of size MxM (for example, 21 x21 for dark-colored eyes and 31 x31 for light-colored eyes) is defined. The mask can be scanned over each of the twenty four sectors, and for each pixel in each sector a value is computed from the region inside the mask centered at that pixel. The value assigned to the pixel is determined as the sum of amplitudes of all spatial frequencies present in the region. In one embodiment, the sum of the amplitudes can be computed by a Fourier transform of the region. If desired, the central 5x5 portion of the Fourier spectrum can be nulled to remove a DC component. The maximum value can then be located in each sector, such that the boundary of its corresponding mask is at least 5 pixels away from the iris image boundary in order to avoid getting close to the pupil margin and other boundary artifacts, such as the eyelid and eyelashes. The "winning" positions and the corresponding blocks are stored for later comparison. Alternatively, the following matrix can be applied. If Gx is the derivative of the block intensity in the x-direction, and Gy is the derivative of the block intensity in the y-direction, then:

$$Z = \begin{bmatrix} \sum Gx^2 & \sum GxGy \\ \sum GxGy & \sum Gy^2 \end{bmatrix}$$

And let $\lambda_1$, $\lambda_2$ be the eigenvalues of the matrix of Z, with $\lambda_2$ being the smaller one, then $\lambda_2$ is the texture strength of the block.

The second image of the eye can also be obtained. (Step 92). In exemplary embodiments, the second image is obtained with imaging assembly 60 of femtosecond laser system 50 prior to forming the incision in the cornea of the patient. The laser imaging assembly may have, for example, a resolution of 680x460 pixels using 256 grayscale levels. The magnification of the laser imaging assembly 60 in relation to the imaging assembly 25 of the measurement system 10 may be different, or may be similar. The eye can be illuminated by a set of infrared LED lights. It should be appreciated, however, that many other imaging devices can be used to obtain different image types.

The sectors in the second image are located and the salient regions that correspond to the salient regions in the reference image are located. (Step 94). For each sector in the second image, a best matching region is located. Optionally, the search is constrained to the matching sector and the two adjacent sectors in the second image, thus limiting possible matches to within 15 degrees, which is a reasonable biological limit for ocular cyclo-rotation. It should be appreciated however, in other embodiments, the range of limiting the possible match may be larger or smaller than 15 degrees. The match between the marker in the reference image and the marker in the second image is evaluated as the sum of absolute errors (after both blocks are made to have zero mean value) for each corresponding region centered at a given pixel. Alternative evaluation methods may also be employed.

Once the corresponding salient regions/markers are located in the second image, an angular displacement for each marker is calculated to estimate a total torsional angle of the eye between the first, reference image and the second image. (Step 96; Figure 9). Additional aspects of torsional registration methods and structures can be understood with reference to U.S. Patent No. 7,044,602, the full disclosure of which is incorporated herein by reference.

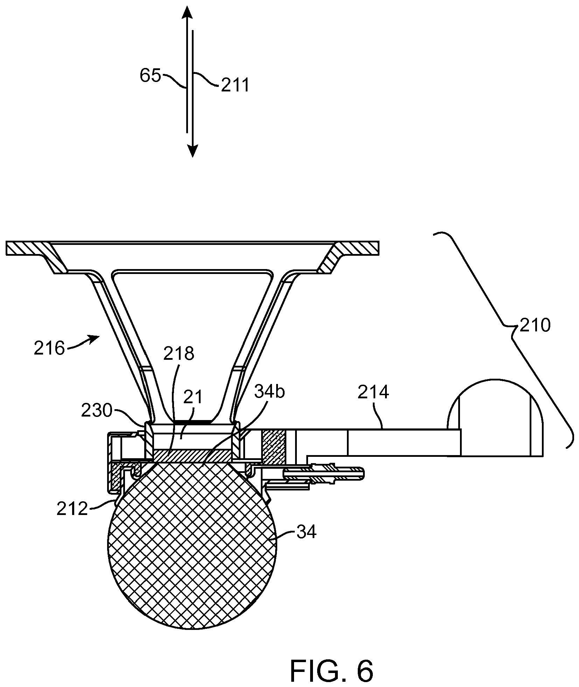

Turning now to FIG. 6, an exemplary embodiment of an ocular fixation device 210, as attached to a human eye 34 during formation of the corneal flap by femtosecond laser system 50 is illustrated in cross-sectional form. The ocular fixation device 210 acts as an interface between the femtosecond laser system 50 and the eye while laser energy 65 is directed from the laser 55 toward the eye, and while an image of the eye 211 is captured by imaging assembly 60 (see FIG. 1).

As more fully described in U.S. Patent No. 7,371,230, fixation device 210 includes a lens cone 216 coupled to an attachment ring 212, thereby coupling a patient's eye 34 to the laser delivery system, by interfacing the two structures together using a gripper/interface 214. An apex ring 230 is inserted into the central opening of the gripper, and an applanation surface 34b of applanation lens 218 makes contact with a presented portion of the anterior surface of the cornea. As the lens cone is lowered into proximity with the cornea, the applanation surface of the lens makes contact with the cornea and applies a pressure to the cornea such that when the lens cone is fully lowered into position, the corneal anterior surface and the applanation surface of the lens are in intimate contact with one another over a substantial portion of the applanation surface. Note that alternative embodiments may use a transparent corneal engagement surface which is curved, so that the cornea may be formed as a concave or convex surface, depending on the shape of the contact surface of the lens. In some instances, applanation of the cornea can distort the eye and affect the reliability of registration based on corneal features. Other alternative embodiments capture an image of the retina. For example, the blood vessel structure of the retina may be used for alignment/registration. Optical coherence tomography (OCT) and Scheimpflug imaging techniques or devices may be used with the fixation device 210 to capture images of the patient's eye 34 while omitting the applanation lens 218.

In use, the attachment ring 212 is placed around the limbus of the eye, i.e., centered about the cornea and the pupillary aperture. The gripper 214 has been previously affixed to the attachment ring 212, such that positioning the ring with respect to the eye also positions the eye with respect to the gripper's central opening, with the pupillary aperture within the gripper's opening. Suction is then applied to the ring in order to attach the ring onto the eye. With the eye so presented and held in place by the attachment ring 212, the lens cone and applanation lens 218 move into proximate contact with the cornea. In the exemplary embodiments, the applanation device is substantially rigidly coupled to the laser delivery system, thus the plane of the applanation surface is characterizable in space with respect to any given focal point of an incident laser beam. With regard to the eye, it should be understood that the applanation lens 218 is able to "float" in the "z" direction and is secured against lateral motion and is accurately disposed in a stable "x,y" plane with respect to the eye.

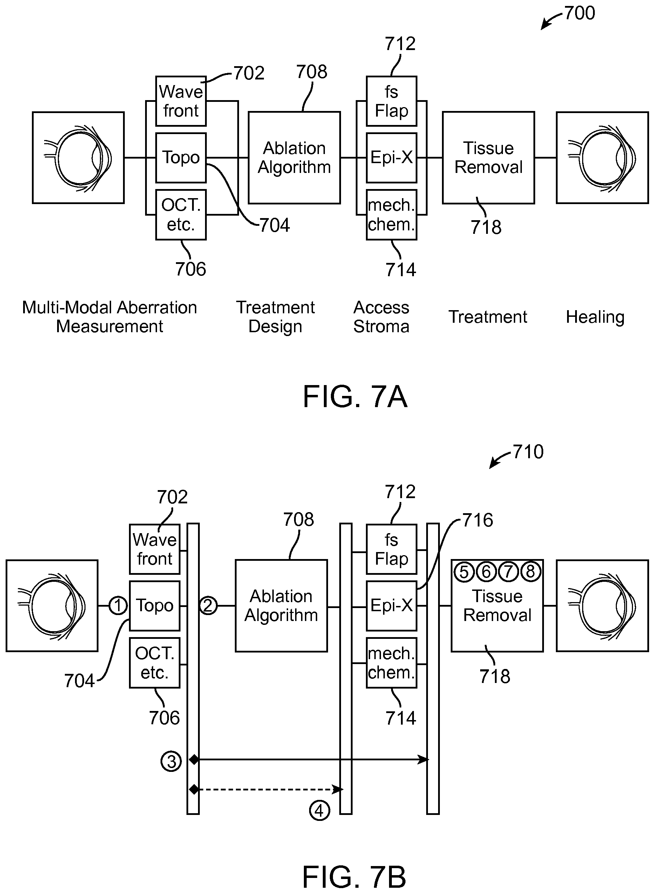

Referring now to FIGs. 7A and 7B, an exemplary laser vision correction process flow 700 suitable for flap registion 710 is schematically illustrated. Multi-modal aberration measurements may be obtained using a wavefront (or other) aberrometer 702, a topographer 704, optical coherence tomography 706, and/or the like. Images of the eye may be obtained in associated with the measurements obtained by one or more of these devices, with eye reference locations optionally being identified in some or all of these images. The optical data from the various measurements can then be registered, optionally using techniques similar to those described above and/or in US Patent No. 7,458,682, entitled "Methods and Devices for Registering Optical Measurement Datasets of an Optical System," the full disclosure of which is incorporated herein by reference. In some embodiments, integrated measurement systems (such as integrated wavefront/topography systems) may facilitate optical data registration. Regardless, autofocus of the image capture system(s) and image analysis 1 may be performed, the wavefront, topographic, and other optical data registered 2, and iris registration data identified 3. The optical data will typically be used to generate an refractive prescription using a processor running software based on an ablation algorithm 708, a femtosecond or other corneal incision pattern calculation algorithm, a corneal collagen remodeling algorithm, or the like to identify an appropriate treatment design or pattern.

Determination of an appropriate incision or other approach for accessing a suitable region of stroma for a particular patient so as to impose the associated corneal reshaping may be based on the shape of the refractive ablation or other refractive therapy. Other factors which may be included in an incision determining calculation might include an epithelial thickness or an epithelial thickness map of the patient, a desired hinge orientation, and/or the like. The stroma can then be accessed by forming the incision, preferably with the incision and flap being registered 4 to the eye by obtaining another image of the eye and using the image registration techniques described above for directing a femtosecond flap cutting laser 712. Alternative embodiments may employ other methods for accessing the stroma, optionally including mechanical or chemical epithelial removal 714 (such as a microkeratome or the like), epithelial removal 716, or the like. Regardless of the specific stroma access technique, automated accessing of the stroma via image registration of the flap 4 or other opening through the epithelium may be employed. The patient may (or may not) be repositioned between measurement of the aberration and accessing of the stroma. Regardless the eye will often move, with that movement being largely compensated for by the image-based flap registration 4.

Before and/or during tissue removal 718, additional images of the eye may be captured. Once again, the patient may or may not be repositioned between stroma access and treatment, but the eye often undergoing at least some movement between initiation of the access process and completion of the treatment process. So as to compensate for that movement, the treatment system may register the prescription with the eye using image processing. Optionally, a third image of the eye may be acquired by the laser eye surgery system, with the processor calculating an X-Y offset 5 (and optionally an X-Y-Z offset) by comparing the third image (including the iris center or the like) to the first image. Similarly, a cyclotorsional offset 6 between the first and third images may be calculated with reference to iris features of the eye in both images. High speed tracking 7 during treatment may then optionally be performed by selective comparison of reference features of the eye in the first and third images, optionally using techniques described in U.S. Patent No. 7,044,602, previously incorporated herein by reference. Alternatively, registration of the laser system and/or tracking of the eye may be performed with reference to the flap, particularly where the flap has been registered with the eye using the methods described above. Optionally, registration of flap and the tissue of the eye may be verified by a comparison between the desired incision (as referenced to the first image) and the image of the incision (as seen in the third image), with the coordinate systems being registered (for example) by reference to the iris centers and corresponding iris features in each of the images of the eye. If appropriate, an X-Y offset and angular offset between the desired and actual flap geometry can be determined. Subsequent images of the eye that encompass the flap can then be used to trach movements of the eye

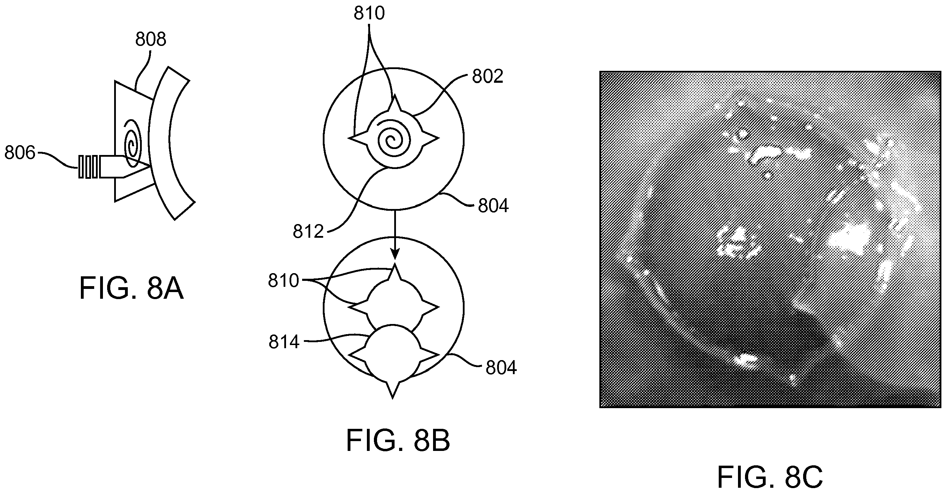

Referring now to FIGs.8A-8C, exemplary flap geometries 802 may be formed in eye 804 by a femtosecond laser 806 through an interface 808. Flap geometry 802 includes rotationally asymmetric features 810 that provide a clear rotationally asymmetric image. While a flap hinge might be used as such a feature in some embodiments, the folding of the flap over the hinge may cover the hinge itself, rendering the angular orientation of that structure less definite that an exposed rotationally symmetric feature that is visible in the image. An image capture device focused on the exposed stroma (rather than on the iris through the incised surface) may allow an outline of the incision to be identified, so as to indicate the flap center, rotationally asymmetric features, and the like, as can be understood with reference to FIG 8C. Comparison of this flap geometry to the desired flap geometry will allow the eye location to be determined (and as subsequent images are captured by the image capture device of the eye treatment system, will also allow movement of the eye to be tracked).

While the above is a complete description of the preferred embodiments of the inventions, various alternatives, modifications, and equivalents may be used. Although the foregoing invention has been described in detail for purposes of clarity of understanding, a variety of changes, adaptations, and modifications may be practiced within the scope of the appended claims.

**Claims**

1. A system for treating an eye having a cornea, pupil and iris, the system comprising:

an ophthalmic diagnostic device (20) having a first image capture device (25) for obtaining a first image of the eye (15A) during a diagnostic procedure;
a femtosecond laser system (55) having a second image capture device (60) for obtaining a second image of the eye (15B) during formation of a laser corneal incision for forming a corneal flap, the corneal incision referenced to the first image;
a processor system (70) coupling the diagnostic device to the laser system, the processor system configured to direct laser energy from the laser system toward the eye during use by comparing the first image with the second image so as to register the corneal incision with the eye, **characterized in that** the processor system is further configured to generate a refractive laser treatment in response to the diagnostic procedure, to determine a desired corneal incision comprising determining a desired corneal flap referenced to the first image, wherein the desired corneal flap has a desired flap center and a desired rotationally asymmetric feature (810) referenced to the first image, wherein the laser corneal incision defines a corneal flap having a flap center and a rotationally asymmetric feature;

and the system further comprises

a refractive reshaping laser system configured to capture a third image, to register the refractive laser treatment with the third image by determining an X-Y offset between the desired flap center referenced to the first image and the flap center in the third image, and to determine an angular offset between a desired flap angle referenced to the first image and the rotationally asymmetric feature in the third image.

2. The system of claim 1, wherein the processor system is configured to determine a geometry of the corneal flap in response to diagnostic data generated by the diagnostic system.

3. The system of claim 1, wherein the processor system is configured to produce target flap location information by processing the images, the target flap location information including X-Y offsets for aligning a center of the iris in the first image to a center of the iris in the second image, and an angular offset for torsionally aligning iris features from the first image with corresponding iris features from the second image.

4. The system of claim 1, wherein the diagnostic device comprises a wavefront aberrometer.

5. The system of claim 1, further comprising an interface (216) having a transparent surface (218) oriented to engage the eye during the corneal incision procedure, wherein the image capture device is oriented so as to obtain the second image through the transparent surface.

6. The system of claim 3, wherein the processor system is configured to direct the refractive laser treatment from the refractive reshaping laser system toward the cornea by comparing the target flap location information to the third image of the eye encompassing the incised flap.

7. The system of any preceding claim, wherein the desired rotationally asymmetric feature is a flap hinge and the rotationally asymmetric feature of the corneal incision is a flap hinge.

**Patentansprüche**

1. System zum Behandeln eines Auges mit Hornhaut, Pupille und Iris, wobei das System umfasst:

eine ophthalmische Diagnostikvorrichtung (20) mit einer ersten Bilderfassungsvorrichtung (25) zum Erhalten eines ersten Bildes des Auges (15A) während eines diagnostischen Verfahrens;
ein Femtosekundenlasersystem (55) mit einer zweiten Bilderfassungsvorrichtung (60) zum Erhalten eines zweiten Bildes des Auges (15B) während der Bildung einer Laser-Hornhautinzision zum Bilden eines Hornhaut-Flaps, wobei die Hornhautinzision sich auf das erste Bild bezog,
ein Prozessorsystem (70), welches das Diagnostiksystem an das Lasersystem koppelt, wobei das Prozessorsystem konfiguriert ist, um während des Gebrauchs Laserenergie aus dem Lasersystem in Richtung des Auges zu lenken, indem das erste Bild mit dem zweiten Bild verglichen wird, um so die Hornhautinzision mit dem Auge zu registrieren,

**dadurch gekennzeichnet, dass** das Prozessorsystem ferner konfiguriert ist, um eine refraktive Laserbehandlung in Reaktion auf das Diagnostikverfahren zu generieren, um eine gewünschte Hornhautinzision zu bestimmen, was das Bestimmen eines gewünschten Hornhaut-Flaps umfasst, welches sich auf das erste Bild bezog, wobei das gewünschte Hornhaut-Flap eine gewünschte Flap-Mitte und ein gewünschtes rotationsasymmetrisches Merkmal (810) aufweist, welches sich auf das erste Bild bezog, wobei die Laser-Hornhautinzision ein Hornhaut-Flap mit einer Flap-Mitte und einem rotationsasymmetrischen Merkmal definiert;
und das System ferner umfasst

ein refraktives Umformungs-Lasersystem, das zum Erfassen eines dritten Bildes konfiguriert ist, um die refraktive Laserbehandlung mit dem dritten Bild zu registrieren, indem ein X-Y-Versatz zwischen der gewünschten Flap-Mitte, die sich auf das erste Bild bezog, umd der Flap-Mitte in dem dritten Bild zu bestimmen, und um einen Winkelversatz zwischen einem gewünschten Flap-Winkel, der sich auf das erste Bild bezog, und dem rotationsasymmetrischen Merkmal in dem dritten Bild zu bestimmen.

**2.** System nach Anspruch 1, wobei das Prozessorsystem konfiguriert ist, um eine Geometrie des Hornhaut-Flaps in Reaktion auf diagnostische Daten zu bestimmen, die durch das diagnostische System generiert wurden.

**3.** System nach Anspruch 1, wobei das Prozessorsystem konfiguriert ist, um Ziel-Flap-Positionsinformationen zu produzieren, indem die Bilder verarbeitet werden, wobei die Ziel-Flap-Positionsinformationen X-Y-Versatze zum Ausrichten einer Mitte der Iris in dem ersten Bild auf eine Mitte der Iris in dem zweiten Bild und einen Winkelversatz zur torsionsbezogenen Ausrichtung von Irismerkmalen aus dem ersten Bild mit entsprechenden Irismerkmalen aus dem zweiten Bild einschließen.

**4.** System nach Anspruch 1, wobei die diagnostische Vorrichtung ein Wellenfront-Aberrometer umfasst.

**5.** System nach Anspruch 1, ferner umfassend ein Interface (216) mit einer transparenten Oberfläche (218), die orientiert ist, um während des Hornhautinzisionsvorgangs in Eingriff mit dem Auge zu sein, wobei die Bilderfassungsvorrichtung so orientiert ist, dass sie das zweite Bild durch die transparente Oberfläche hindurch erhält.

**6.** System nach Anspruch 3, wobei das Prozessorsystem konfiguriert ist, um die refraktive Laserbehandlung von dem refraktiven Umformungs-Lasersystem in Richtung der Hornhaut zu lenken, indem die Ziel-Flap-Positionsinformationen mit dem dritten Bild des Auges verglichen werden, welches den inzidierten Flap umschließt.

**7.** System nach einem der vorhergehenden Ansprüche, wobei das gewünschte rotationsasymmetrische Merkmal ein Flap-Hinge (Klappstelle des Flaps) ist und das rotationsasymmetrische Merkmal der Hornhautinzision ein Flap-Hinge ist.

**Revendications**

**1.** Système destiné à traiter un oeil ayant une cornée, une pupille et un iris, le système comprenant :

un dispositif de diagnostic ophtalmique (20) ayant un premier dispositif de capture d'image (25) destiné à obtenir une première image de l'oeil (15A) pendant une procédure de diagnostic ;
un système laser femtoseconde (55) ayant un deuxième dispositif de capture d'image (60) destiné à obtenir une deuxième image de l'oeil (15B) pendant la formation d'une incision cornéenne au laser pour former un volet cornéen, l'incision cornéenne étant repérée dans la première image ;
un système de processeur (70) couplant le dispositif de diagnostic au système laser, le système de processeur étant configuré pour diriger l'énergie laser issue du système laser vers l'oeil à l'usage en comparant la première image avec la deuxième image de manière à aligner l'incision cornéenne avec l'oeil, **caractérisé en ce que** le système de processeur est également configuré pour générer un traitement laser réfractif en réponse à la procédure de diagnostic, pour déterminer une incision cornéenne souhaitée comprenant la détermination d'un volet cornéen souhaité repéré dans la première image, le volet cornéen souhaité ayant un centre de volet souhaité et une caractéristique asymétrique en rotation souhaitée (810) repérés dans la première image, l'incision cornéenne au laser définissant un volet cornéen ayant un centre de volet et une caractéristique asymétrique en rotation ;

et le système comprenant en outre un système laser de refaçonnage réfractif configuré pour capturer une troisième image, pour aligner le traitement laser réfractif avec la troisième image en déterminant un décalage X-Y entre le centre de volet souhaité repéré dans la première image et le centre de volet dans la troisième image, et pour déterminer un décalage angulaire entre un angle de volet souhaité repéré dans la première image et la caractéristique asymétrique en rotation dans la troisième image.

**2.** Système de la revendication 1, dans lequel le système de processeur est configuré pour déterminer une géométrie du volet cornéen en réponse à des données de diagnostic générées par le système de diagnostic.

**3.** Système de la revendication 1, dans lequel le système de processeur est configuré pour générer des informations d'emplacement de volet cible en traitant les images, les informations d'emplacement de volet cible comportant des décalages X-Y pour aligner un centre de l'iris dans la première image sur un centre de l'iris dans la deuxième image, et un décalage angulaire pour aligner en torsion des caractéristiques de l'iris issues de la première image avec des caractéristiques correspondantes de l'iris issues de la deuxième image.

**4.** Système de la revendication 1, dans lequel le dispositif de diagnostic comprend un aberromètre de front d'onde.

**5.** Système de la revendication 1, comprenant en outre une interface (216) ayant une surface transparente (218) orientée pour engager l'oeil pendant la procédure d'incision cornéenne, le dispositif de capture d'image étant orienté de manière à obtenir la deuxième image à travers la surface transparente.

6.  Système de la revendication 3, dans lequel le système de processeur est configuré pour diriger le traitement laser réfractif depuis le système laser de refaçonnage réfractif vers la cornée en comparant les informations d'emplacement de volet cible à la troisième image de l'oeil englobant le volet incisé.

7.  Système d'une quelconque revendication précédente, dans lequel la caractéristique asymétrique en rotation souhaitée est une charnière de volet et la caractéristique asymétrique en rotation de l'incision cornéenne est une charnière de volet.

FIG. 1

FIG. 1A

FIG. 2A

FIG. 2B

| Generating a first image of the eye | ─300 |

| Determine a corneal flap geometery with respect to the first image | ─305 |

| Generating a second image of the eye | ─310 |

| Comparing the first image to the second image | ─315 |

| Registering the corneal flap geometry of the first image to the second image | ─320 |

FIG. 3

| Generating a first image of the eye | 400 |

| Calculate a reference location of the eye | 405 |

| Determine a desired corneal flap geometry with respect to the reference location | 410 |

| Registering the desired corneal flap geometry to the eye | 415 |

FIG. 4

START

Obtain a first image of an eye — 80

Locate the pupil and radius in the first image of the eye — 82

Extract a fixed or variable width iris ring — 84

Unwrap the iris ring and divide into a fixed number of sectors — 86

Locate a salient region in each sector in the first image of the eye — 88

Extract the properties of each of the salient region — 90

Obtain a second image of the eye — 92

Locate the salient region of the second image of the eye — 94

Estimate the angular displacement for each of the salient regions and estimate the total torsional angle estimation — 96

END

FIG. 5

65 211

216 218 21 34b 214 210

230

212 34

# FIG. 6

FIG. 7A

FIG. 7B

FIG. 8C

FIG. 8B

FIG. 8A

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61243654 B
- US 20030223037 A
- US 6322216 B
- US 7044602 B
- US 7371230 B
- US 7458682 B